# EUROPÄISCHE PATENTANMELDUNG

(11) **EP 3 489 692 A1**
(43) Veröffentlichungstag der Anmeldung: **29.05.2019**
(21) Anmeldenummer: 17204000.8
(22) Anmeldetag: 28.11.2017
(51) Int. Cl.: G01N 33/86

(54) **PROTHROMBINZEIT-REAGENZ ENTHALTEND EINEN EISENCHELATOR**

(71) Anmelder: Siemens Healthcare Diagnostics Products GmbH, 35041 Marburg (DE)
(72) Erfinder: Pilgrim, Sabine, 35037 Marburg (DE); Wissel, Thomas, 35094 LAHNTAL (DE); Zander, Norbert, 35039 Marburg (DE)

(57) **Zusammenfassung**

Die vorliegende Erfindung liegt auf dem Gebiet der Gerinnungsdiagnostik und betrifft ein Prothrombinzeit-Reagenz auf Basis von rekombinantem oder nativem Gewebefaktorprotein und Phospholipiden, welches durch die Zugabe des Eisenchelators Deferoxamin stabilisiert werden kann.

## Beschreibung

Die vorliegende Erfindung liegt auf dem Gebiet der Gerinnungsdiagnostik und betrifft ein Prothrombinzeit-Reagenz auf Basis von rekombinantem oder nativem Gewebefaktorprotein und Phospholipiden, welches durch die Zugabe des Eisenchelators Deferoxamin stabilisiert werden kann.

Das Gewebefaktorprotein (kurz: Gewebefaktor oder Thromboplastin, engl. tissue factor) ist ein Transmembranprotein mit essentieller Bedeutung für die Blutgerinnung. Es wird von Zellen exprimiert, die normalerweise nicht mit fließendem Blut in Kontakt stehen, wie z.B. von Zellen im Subendothel (Glatte Muskulatur) und von Zellen, die Blutgefäße umgeben (z.B. Fibroblasten). Im Falle einer Blutgefäßschädigung geraten die Gewebefaktorprotein-exprimierenden Zellen jedoch in Kontakt mit Faktor VII, einem prokoagulatorischen Blutgerinnungsfaktor, der im Blut zirkuliert. In Gegenwart von Calcium bilden Gewebefaktorprotein und Faktor VII einen Komplex, und die Aktivität von Faktor VII wird vertausendfacht (F VII > F VIIa). Der Komplex aus Gewebefaktorprotein und Faktor VIIa katalysiert in Gegenwart von Phospholipiden und Calcium die Umwandlung des inaktiven Blutgerinnungsfaktors Faktor X in aktivierten Faktor Xa und beschleunigt so den Gerinnungsprozess. Zusammen mit Faktor VII bildet der Gewebefaktor den sogenannten extrinsischen Weg der Blutgerinnung, durch den eine Verletzung der Blutgefäße durch schnellstmögliche Blutgerinnung unterbunden werden soll.

In der Gerinnungsdiagnostik wurden verschiedene in vitro-Testverfahren entwickelt, mit deren Hilfe bestimmt werden kann, ob das Blut oder Plasma eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Zur Untersuchung verschiedener Teilfunktionen der Blutgerinnung, insbesondere zur Untersuchung des extrinsischen Systems der Blutgerinnung, wird Gewebefaktorprotein als Aktivator verwendet. Die bekannteste Anwendung von Gewebefaktorprotein als Gerinnungsaktivator ist der sogenannte Quick-Test zur Bestimmung der Prothrombinzeit (PT) (synonym: Thromboplastinzeit). Im Quick-Test und seinen Varianten wird üblicherweise eine Plasmaprobe mit einer Mischung von Gewebefaktorprotein, Phospholipiden und Calciumionen vermischt, und es wird die Zeit vom Zeitpunkt des Vermischens bis zur fassbaren Fibrinbildung in Sekunden gemessen. In Gerinnungstesten, bei denen chromogene Substrate verwendet werden, wird alternativ die Zeit vom Zeitpunkt des Vermischens bis zum Erreichen einer bestimmten Absorptionsänderung gemessen. Gewebefaktorprotein wird auch in anderen Testverfahren eingesetzt, die nicht der Bestimmung einer Gerinnungszeit dienen, sondern der Bestimmung einzelner Komponenten des Gerinnungssystems, wie z.B. des endogenen Thrombinpotenzials (ETP) (EP-A2-420332). Prinzipiell kann Gewebefaktorprotein bei allen Testen verwendet werden, die sich mit Komponenten der extrinsischen Gerinnung beschäftigen.

Das Prothrombinzeit-Reagenz (Gewebefaktor-Reagenz, PT-Reagenz) hat eine zentrale Bedeutung für den jeweiligen Test. Üblicherweise enthält ein Prothrombinzeit-Reagenz den Gewebefaktor zusammen mit gerinnungsaktiven Phospholipiden. Das Gewebefaktorprotein wird entweder als Gewebeextrakt aus unterschiedlichen Organen (z.B. Hirn, Plazenta, Lunge) verschiedener Spezies (z.B. Kaninchen, Mensch, Rind) gewonnen oder rekombinant hergestellt. Im Stand der Technik sind zahlreiche Methoden zur Gewinnung von Gewebefaktorprotein und zur Herstellung von Prothrombinzeit-Reagenzien bekannt, und eine Vielzahl von Prothrombinzeit-Reagenzien ist kommerziell erhältlich.

Zurzeit werden die meisten käuflichen Prothrombinzeit-Reagenzien in gefriergetrockneter Form gehandelt und müssen daher vor Gebrauch in einem Rekonstitutionsmedium, z.B. in destilliertem Wasser oder einer Pufferlösung aufgelöst werden. Der Grund hierfür ist die mangelnde Stabilität der Reagenzien im flüssigen Zustand. Der Nachteil von Reagenzien, die in gefriergetrockneter Form bereit gestellt werden, besteht nicht nur darin, dass Hersteller und Anwender zusätzliche zeit- und kostenintensive Verfahrensschritte durchführen müssen (Lyophilisation und Rekonstitution), sondern auch darin dass diese zusätzlichen Maßnahmen das Risiko bergen, dass es dabei zu Fehlern kommt, die die Qualität des Reagenzes beeinträchtigen können. Wünschenswert sind daher flüssige, gebrauchsfertige Reagenzformulierungen. Ein Problem bei der Bereitstellung flüssiger Prothrombinzeit-Reagenzien ist jedoch ihre mangelnde Stabilität. Unter der Stabilität eines Prothrombinzeit-Reagenzes kann z.B. die Beibehaltung der Prothrombinzeit für ein definiertes Plasma, z.B. ein Normalplasma, über die Lagerungzeit verstanden werden. Idealerweise sollte ein Prothrombinzeit-Reagenz über die Dauer seiner Lagerung oder seines Gebrauchs seine Spezifikationen beibehalten, beziehungsweise im günstigsten Fall die Eigenschaften und Charakteristika wie zum Zeitpunkt seiner Produktion.

Im Stand der Technik sind verschiedene Strategien zur Stabilisierung flüssiger Prothrombinzeit-Reagenzien beschrieben. In EP-A2-942284 wird ein flüssiges, auf rekombinantem Gewebefaktor basierendes Prothrombinzeit-Reagenz beschrieben, das durch die kombinierte Zugabe von Ascorbinsäure und einem Serumalbumin stabilisiert wird. In US 3,522,148 wird ein flüssiges, aus Gewebe extrahiertes (natürliches) Gewebefaktorprotein enthaltendes Prothrombinzeit-Reagenz beschrieben, das durch Zugabe bestimmter Natrium- oder Calciumsalze stabilisiert wird. In EP-A1-585987 wird ein anderes flüssiges, auf natürlichem Gewebefaktorprotein basierendes Prothrombinzeit-Reagenz beschrieben, das durch die Zugabe verschiedener Stabilisatoren, wie Albumin oder Polyethylenglykol, sowie verschiedener antimikrobiell wirksamer Substanzen, wie Natriumazid oder Antibiotika, stabilisiert wird. In EP-A2-524803 wird ein Verfahren zur Herstellung eines Prothrombinzeit-Reagenzes beschrieben, bei dem ein Metallionenchelator, insbesondere EDTA oder EGTA, bei der Extraktion des Gewebefaktors eingesetzt wird und im fertigen Reagenz enthalten sein kann. Die von dem Metallionenchelator gebundene Menge an Calciumionen wird durch Zugabe von zusätzlichem Calciumsalz ersetzt.

In US 2017/0234895 A1 ist ein zweiteiliges Kit zur Herstellung eines gebrauchsfertigen, flüssigen Prothrombinzeit-Reagenzes beschrieben, das in einem ersten Gefäß ein erste Lösung enthaltend Gewebefaktorprotein und Phospholipide enthält und in einem zweiten Gefäß eine Calciumchloridlösung enthält. In der ersten Lösung ist zum Zwecke der Stabilisierung ein Calciumchelator enthalten. Die beiden Lösungen werden erst kurz vor Gebrauch vom Anwender miteinander vermischt. Zwar kann hier seitens des Herstellers auf die Lyophilisation und seitens des Anwenders auf die Rekonstitutuion eines lyophilisierten Reagenzes verzichtet werden, allerdings muss der Anwender immer noch die beiden Flüssigkeiten miteinander vermischen, um das gebrauchsfertige Prothrombinzeit-Reagenz zu erhalten.

Der vorliegenden Erfindung lag also die Aufgabe zugrunde, ein im flüssigen Zustand langzeitstabiles, gebrauchsfertiges Prothrombinzeit-Reagenz, das mindestens Gewebefaktorprotein und Phospholipide enthält und das optional auch Calciumionen enthalten kann, bereit zu stellen.

Es wurde gefunden, dass die Zugabe eines Eisenchelators, insbesondere eines Eisenchelators aus der Gruppe der Siderophore und darunter insbesondere die Zugabe von Deferoxamin die Stabilität einer wässrigen Lösung, die Gewebefaktor und Phospholipide und optional Calciumionen enthält, erhöht. Erfindungsgemäß stabilisierte und im flüssigen Zustand gelagerte Prothrombinzeit-Reagenzien liefern noch nach 10-wöchiger Lagerung bei +37 °C Prothrombinzeit- (PT-) Messergebnisse, die weniger als 20 % von dem PT-Messergebnis zu Beginn der Laufzeit abweichen. Eine forcierte Lagerung bei +37 °C über 10 Wochen dient der schnellen Abschätzung, ob eine Lagerungsstabilität bei +2 °C bis +8 °C über einen Zeitraum von 12 oder mehr Monaten erwartet werden kann. Liefert ein stabilisiertes Prothrombinzeit-Reagenz bei einer Lagerung bei +37 °C über einen Zeitraum von 10 Wochen Prothrombinzeit- (PT-) Messergebnisse, die weniger als 20 % von dem PT-Messergebnis zu Beginn der Laufzeit abweichen, kann erwartet werden, dass das Reagenz bei einer Lagerungstemperatur von +2 °C bis +8 °C über einen Zeitraum von 12 oder mehr Monaten stabil ist.

Gegenstand der vorliegenden Erfindung ist also ein Prothrombinzeit-Reagenz enthaltend Gewebefaktorprotein und Phospholipide, das ferner einen Eisenchelator aus der Gruppe der Siderophore enthält.

Siderophore sind Eisen-bindende Chelatbildner, die von Mikroorganismen wie Bakterien und Pilzen und von Pflanzen gebildet werden und eine hohe Affinität für Eisen-III-Ionen (Fe³⁺-Ionen) aufweisen.

Bevorzugterweise enthält ein erfindungsgemäßes Prothrombinzeit-Reagenz den Eisenchelator Deferoxamin (synonym: Desferrioxamin; Handelsname: Desferal). Deferoxamin ist vorzugsweise in einer Konzentration von 5 bis 1500 mg/L in dem Reagenz enthalten.

In einer besonders bevorzugten Ausführungsform enthält das Prothrombinzeit-Reagenz ferner Calciumionen. Zu diesem Zweck kann das Prothrombinzeit-Reagenz Calciumchlorid enthalten, vorzugsweise in einer Konzentration von 5 bis 500 mmol/L.

Das in dem Prothrombinzeit-Reagenz enthaltene Gewebefaktorprotein ist vorzugsweise ausgewählt aus der Gruppe humanes oder tierisches (z.B. Kaninchen, Rind etc.) rekombinantes Gewebefaktorprotein und natürliches humanes oder tierisches Gewebefaktorprotein aus einem Gewebextrakt (z.B. aus Hirn, Plazenta, Lunge etc.).

Die Phospholipide können aus natürlichen und/oder synthetischen Quellen stammen.

Auch wenn der besondere Vorteil der vorliegenden Erfindung darin besteht, dass ein erfindungsgemäßes Prothrombinzeit-Reagenz als gebrauchsfertiges Flüssigreagenz gelagert werden kann, so ist es jedoch selbstverständlich auch möglich, dass das Reagenz als in Wasser oder Puffer rekonstituierbares Lyophilisat bereitgestellt wird.

Ein weiterer Gegenstand der vorliegenden Erfindung ist ein Verfahren zur Herstellung eines flüssigen Prothrombinzeit-Reagenzes, wobei eine Flüssigkeit enthaltend Gewebefaktorprotein, Phospholipide und mindestens einen Eisenchelator aus der Gruppe der Siderophore, bevorzugterweise Deferoxamin, bereit gestellt wird und in ein Reagenzgefäß abgefüllt wird, ohne dass diese Flüssigkeit lyophilisiert wird.

Noch ein weiterer Gegenstand der vorliegenden Erfindung ist ein Verfahren zur Stabilisierung eines Prothrombinzeit-Reagenzes enthaltend Gewebefaktorprotein und Phospholipide und optional Ca²⁺-Ionen, indem dem Reagenz mindestens ein Eisenchelator aus der Gruppe der Siderophore, vorzugsweise Deferoxamin zugesetzt wird.

Ein anderer Gegenstand der vorliegenden Erfindung ist die Verwendung eines erfindungsgemäßen Prothrombinzeit-Reagenzes in einem in vitro-Verfahren zur Bestimmung eines Gerinnungsparameters in einer Patientenprobe, inbesondere zur Bestimmung eines Gerinnungsparameters aus der Gruppe Prothrombinzeit (PT, auch Quick-Test) und deren Varianten, von der Prothrombinzeit abgeleitetes Fibrinogen und Endogenes Thrombinpotenzial (ETP). Das erfindungsgemäße Reagenz eignet sich zur Verwendung als Aktivator der Gerinnungskaskade, beispielsweise in Testverfahren, die auf der Detektion eines Fibringerinnsels beruhen wie auch in chromogenen oder fluorogenen Testverfahren.

Die Erfindung betrifft ferner ein Verfahren zur Bestimmung eines Gerinnungsparameters in einer Plasmaprobe, wobei die Plasmaprobe mit einem erfindungsgemäßen Prothrombinzeit-Reagenz zu einem Reaktionsgemisch vermischt wird und der Gerinnungsparameter in dem Reaktionsgemisch bestimmt wird. Bei dem Gerinnungsparameter kann es sich beispielsweise um die Prothombinzeit oder das endogene Thrombinpotenzial einer Probe, beispielsweise einer humanen Vollblut- oder Plasmaprobe handeln.

Zur Bestimmung der Prothombinzeit wird vorzugsweise die Absorptionsänderung des Reaktionsgemisches photometrisch gemessen, und es wird die Zeit vom Zeitpunkt des Vermischens des erfindungsgemäßen Prothrombinzeit-Reagenzes mit der Plasmaprobe bis zur Erreichung eines Schwellwertes bestimmt.

Zur Ableitung der Fibrinogenkonzentration aus der Prothrombinzeitbestimmung ("abgeleitetes Fibrinogen") wird die Absorptionszunahme des Reaktionsgemisches photometrisch gemessen; diese korreliert mit der Fibrinogenkonzentration.

### FIGURENBESCHREIBUNG

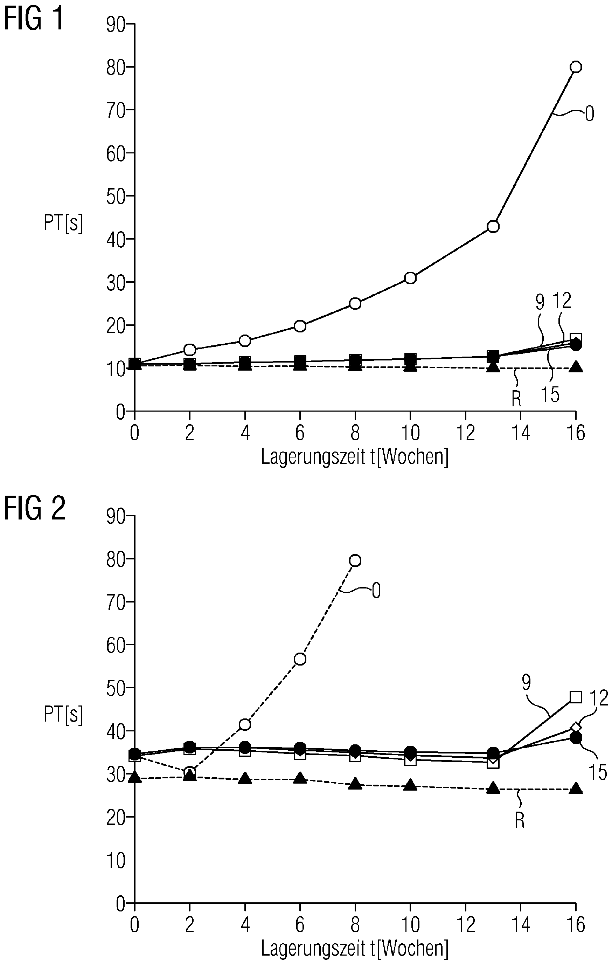

**Figur 1** zeigt die Veränderung der Prothrombinzeiten in Sekunden (PT [s]) des normalen Kontrollplasmas Ci-Trol 1 mit den mit Deferoxamin stabilisierten PT-Reagenzien (9 = 9 mg/mL Deferoxamin; 12 = 12 mg/mL Deferoxamin; 15 = 15 mg/mL Deferoxamin) im Vergleich zu dem nicht stabilisierten PT-Reagenz ohne Deferoxamin (0 = 0 mg/mL Deferoxamin) und zu dem lyophilisiert gelagerten und zum jeweiligen Zeitpunkt frisch gelösten Referenzreagenz (R) über die Zeit (t) in Wochen. Die Reagenzien wurden bei einer Temperatur von 37 °C gelagert.
**Figur 2** zeigt die Veränderung der Prothrombinzeiten in Sekunden (PT [s]) des abnormalen Kontrollplasmas Ci-Trol 2 mit den mit Deferoxamin stabilisierten PT-Reagenzien (9 = 9 mg/mL Deferoxamin; 12 = 12 mg/mL Deferoxamin; 15 = 15 mg/mL Deferoxamin) im Vergleich zu dem nicht stabilisierten PT-Reagenz ohne Deferoxamin (0 = 0 mg/mL Deferoxamin) und zu dem lyophilisiert gelagerten und zum jeweiligen Zeitpunkt frisch gelösten Referenzreagenz (R) über die Zeit (t) in Wochen. Die Reagenzien wurden bei einer Temperatur von 37 °C gelagert.

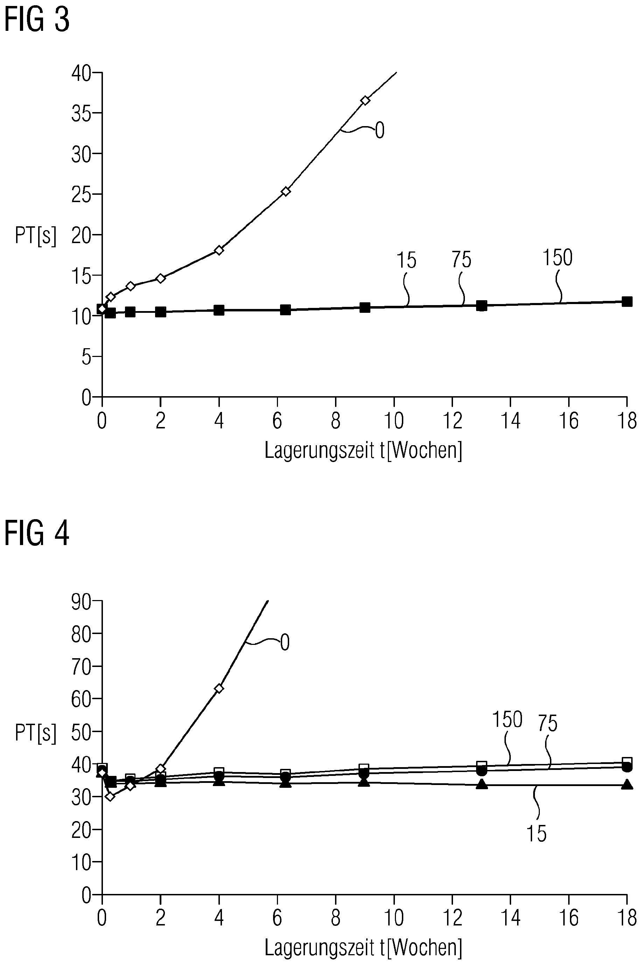

**Figur 3** zeigt die Veränderung der Prothrombinzeiten in Sekunden (PT [s]) des normalen Kontrollplasmas Ci-Trol 1 mit den mit Deferoxamin stabilisierten PT-Reagenzien (15 = 15 mg/mL Deferoxamin; 75 = 75 mg/mL Deferoxamin; 150 = 150 mg/mL Deferoxamin) im Vergleich zu dem nicht stabilisierten PT-Reagenz ohne Deferoxamin (0 = 0 mg/mL Deferoxamin) über die Zeit (t) in Wochen. Die Reagenzien wurden bei einer Temperatur von 37 °C gelagert.
**Figur 4** zeigt die Veränderung der Prothrombinzeiten in Sekunden (PT [s]) des abnormalen Kontrollplasmas Ci-Trol 2 mit den mit Deferoxamin stabilisierten PT-Reagenzien (15 = 15 mg/mL Deferoxamin; 75 = 75 mg/mL Deferoxamin; 150 = 150 mg/mL Deferoxamin) im Vergleich zu dem nicht stabilisierten PT-Reagenz ohne Deferoxamin (0 = 0 mg/mL Deferoxamin) über die Zeit (t) in Wochen. Die Reagenzien wurden bei einer Temperatur von 37 °C gelagert.

Die folgenden Ausführungsbeispiele dienen der Veranschaulichung der vorliegenden Erfindung und sind nicht als Einschränkung zu verstehen.

### BEISPIEL 1: Bestimmung der Stabilität eines flüssigen, mit Deferoxamin stabilisierten Prothrombinzeit-Reagenzes

Zum Zeitpunkt t₀ wurde einem flüssigen Prothrombinzeit-Reagenz, das rekombinantes humanes Gewebefaktorprotein, synthetische Phospholipide und Calciumionen enthielt, in verschiedenen Ansätzen Deferoxamin (Deferoxamine mesylate salt, Sigma-Aldrich Chemie GmbH, Steinheim, Deutschland) in der angegebenen Endkonzentration zugesetzt:
1. 0 mg/L Deferoxamin,
2. 9 mg/L Deferoxamin,
3. 12 mg/L Deferoxamin,
4. 15 mg/L Deferoxamin,
5. 75 mg/L Deferoxamin,
6. 150 mg/L Deferoxamin.

Die verschiedenen Reagenzien wurden in einem automatischen Prothrombinzeit-Test (PT-Test) in einem Sysmex® CA-1500-Analysegerät (Sysmex Corp., Kobe, Japan) eingesetzt. Als Proben wurden folgende definierte Kontrollplasmen eingesetzt:
- Dade® Ci-Trol® Coagulation Control Level 1 (Ci-Trol 1) Kontrollplasma; normale Kontrolle für die Bestimmung der Prothrombinzeit; die Ergebnisse sind mit denen mit frischem normalem Citratplasma vergleichbar;
- Dade® Ci-Trol® Coagulation Control Level 2 (Ci-Trol 2) Kontrollplasma; abnormale Kontrolle für die Bestimmung der Prothrombinzeit wie sie bei Gerinnungsstörungen zu beobachten sind; die Gerinnungszeiten sind gegenüber denen mit frischem normalem Citratplasma verlängert.

Probe und Reagenz wurden jeweils auf 37 °C vorgewärmt und schließlich vermischt. Durch die Zugabe des Reagenzes wurde der Gerinnungsvorgang ausgelöst, und es wurde die Zeit bis zur Bildung des Fibringerinnsels gemessen (Prothrombinzeit in Sekunden).

Zur Bestimmung der Langzeitstabilität der verschiedenen Reagenzien wurden die Reagenzien in flüssigem Zustand bei +37 °C über einen Zeitraum von 16-18 Wochen gelagert. Etwa zweiwöchentlich wurden Proben der Reagenzien entnommen, und es wurde die Prothrombinzeit desselben Referenzplasmas bestimmt. Als Referenzreagenz wurde zu jedem Zeitpunkt ein lyophilisiertes Prothrombinzeit-Reagenz (Dade® Innovin® Reagenz, Siemens Healthcare Diagnostics Prodcts GmbH, Deutschland) mit destilliertem Wasser frisch rekonstituiert und gemessen.

In Figur 1 (Ci-Trol 1 als Probe) und Figur 2 (Ci-Trol 2 als Probe) sind die Prothrombinzeiten der verschiedenen Prothrombinzeit-Reagenzien (0, 12, 15 mg/L Deferoxamin) über einen Zeitraum von 16 Wochen bei +37 °C gezeigt. Stabile Prothrombinzeiten über einen Zeitraum von mindestens 10 Wochen mit Abweichungen von weniger als 20 % gegenüber dem Ausgangswert zum Zeitpunkt t₀ wurden nur mit dem lyophilisierten und jeweils frisch gelösten Referenzreagenz (R) und mit den erfindungsgemäßen, mit Deferoxamin stabilisierten Prothrombinzeit-Reagenzien erreicht. Das flüssig gelagerte Reagenz ohne Deferoxamin lieferte spätestens nach 4 Wochen nur noch stark abweichende Prothrombinzeiten.

In Figur 3 (Ci-Trol 1 als Probe) und Figur 4 (Ci-Trol 2 als Probe) sind die Prothrombinzeiten der verschiedenen Prothrombinzeit-Reagenzien (15, 75, 150 mg/L Deferoxamin) über einen Zeitraum von 18 Wochen bei +37 °C gezeigt. Stabile Prothrombinzeiten über einen Zeitraum von mindestens 10 Wochen mit Abweichungen von weniger als 20 % gegenüber dem Ausgangswert zum Zeitpunkt t₀ wurden nur mit den erfindungsgemäßen, mit Deferoxamin stabilisierten Prothrombinzeit-Reagenzien erreicht. Das flüssig gelagerte Reagenz ohne Deferoxamin lieferte spätestens nach 4 Wochen nur noch stark abweichende Prothrombinzeiten.

## Patentansprüche

1. Prothrombinzeit-Reagenz enthaltend Gewebefaktorprotein und Phospholipide **dadurch gekennzeichnet, dass** das Reagenz ferner einen Eisenchelator aus der Gruppe der Siderophore enthält.

2. Prothrombinzeit-Reagenz gemäß Anspruch 1, das Deferoxamin enthält.

3. Prothrombinzeit-Reagenz gemäß Anspruch 2, das Deferoxamin in einer Konzentration von 5 bis 1500 mg/L enthält.

4. Prothrombinzeit-Reagenz gemäß einem der vorhergehenden Ansprüche, das ferner Ca²⁺-Ionen enthält.

5. Prothrombinzeit-Reagenz gemäß Anspruch 4, das 5 bis 500 mmol/L Calciumchlorid enthält.

6. Prothrombinzeit-Reagenz gemäß einem der vorhergehenden Ansprüche, wobei das Gewebefaktorprotein ausgewählt ist aus der Gruppe humanes oder tierisches rekombinantes Gewebefaktorprotein und natürliches humanes oder tierisches Gewebefaktorprotein aus einem Gewebextrakt.

7. Verfahren zur Herstellung eines flüssigen Prothrombinzeit-Reagenzes enthaltend Gewebefaktorprotein und Phospholipide und optional Ca²⁺-Ionen **dadurch gekennzeichnet, dass** eine Flüssigkeit enthaltend Gewebefaktorprotein, Phospholipide und mindestens einen Eisenchelator aus der Gruppe der Siderophore, bevorzugterweise Deferoxamin, bereit gestellt wird und die Flüssigkeit in ein Reagenzgefäß abgefüllt wird, ohne dass diese Flüssigkeit lyophilisiert wird.

8. Verfahren zur Stabilisierung eines Prothrombinzeit-Reagenzes enthaltend Gewebefaktorprotein und Phospholipide und optional Ca²⁺-Ionen **dadurch gekennzeichnet, dass** dem Reagenz mindestens ein Eisenchelator aus der Gruppe der Siderophore, vorzugsweise Deferoxamin zugesetzt wird.

9. Verwendung eines Prothrombinzeit-Reagenzes gemäß einem der Ansprüche 1-6 in einem Verfahren zur Bestimmung eines Gerinnungsparameters in einer Plasmaprobe.

10. Verfahren zur Bestimmung eines Gerinnungsparameters in einer Plasmaprobe **dadurch gekennzeichnet, dass** die Plasmaprobe mit einem Prothrombinzeit-Reagenz gemäß einem der Ansprüche 1-6 zu einem Reaktionsgemisch vermischt wird und der Gerinnungsparameter in dem Reaktionsgemisch bestimmt wird.

11. Verfahren gemäß Anspruch 10 zur Bestimmung eines Gerinnungsparameters aus der Gruppe Prothombinzeit, abgeleitetes Fibrinogen und endogenes Thrombinpotenzial.

12. Verfahren gemäß Anspruch 10 zur Bestimmung der Prothombinzeit, wobei die Absorptionsänderung des Reaktionsgemisches photometrisch gemessen wird und die Zeit vom Zeitpunkt des Vermischens des Prothrombinzeit-Reagenzes mit der Plasmaprobe bis zur Erreichung eines Schwellwertes bestimmt wird.
